# FASCICULE DE BREVET EUROPEEN

(11) **EP 2 181 709 B1**
(45) Date de publication et mention de la délivrance du brevet: **29.01.2014**
(21) Numéro de dépôt: 09306019.2
(22) Date de dépôt: 27.10.2009
(51) Int. Cl.: A61K 31/395, A61K 31/192, A61K 45/06, A61P 25/04

(54) **Combinaison synergique de composés analgésiques**
Synergistische Kombination von analgetischen Verbindungen
Synergic combination of analgesic compounds

(30) Priorité: 29.10.2008 FR 0857363
(43) Date de publication de la demande: 05.05.2010
(73) Titulaire: Biocodex, 94250 Gentilly (FR)
(72) Inventeur: Girard, Philippe, 60280, MARGNY-LES-COMPIEGNE (FR); Le Guern, Marie-Emmanuelle, 60200, COMPIEGNE (FR); Berthon-Cedille, Laurence, 60490, RICQUEBOURG (FR); Gillardin, Jean-Marie, 60680, JONQUIERES (FR); Hublot, Bernard, 60200, COMPIEGNE (FR)
(74) Mandataire: Vial, Lionel

(56) Documents cités:
- WO-A-2005/060957
- GUERRA-NARDUCCI P; LIOTTO R; ESPOSITO P; MARCIANO M: "Association of Nefopan and Buprenorphine in postsurgical pain in thoracic surgery" RASSEGNA INTERNAZIONALE DI CLINICA E TERAPIA, vol. 73, no. 11, 1993, pages 488-494, XP009115388
- GOSSET S; COURSANGE F; CARPENTIER J P; MALGRAS G; AUBERT M; VINCENT M: "Valium-Acupan combination - its value at an isolated health center" MÉDECINE TROPICALE : REVUE DU CORPS DE SANTÉ COLONIAL, vol. 46, no. 2, avril 1986 (1986-04), pages 195-197, XP009115389
- MANOIR DU B ET AL: "RANDOMIZED PROSPECTIVE STUDY OF THE ANALGESIC EFFECT OF NEFOPAM AFTER ORTHOPAEDIC SURGERY" BRITISH JOURNAL OF ANAESTHESIA, BJM PUBLISHING GROUP, LONDON, GB, vol. 91, no. 6, 1 décembre 2003 (2003-12-01), pages 836-841, XP008046817 ISSN: 0007-0912
- GIRARD ET AL: "Nefopam and ketoprofen synergy in rodent models of antinociception" EUROPEAN JOURNAL OF PHARMACOLOGY, ELSEVIER BV, NL, vol. 584, no. 2-3, 14 février 2008 (2008-02-14), pages 263-271, XP022577317 ISSN: 0014-2999

## Description

### Domaine de l'invention

La présente invention concerne une composition pharmaceutique comprenant des composés ayant une action synergique dans la prévention ou le traitement de la douleur.

### Arrière plan technique

Le néfopam est le principe actif de l'Acupan®. II s'agit d'un antalgique central non opioïde, de la famille des benzoxazocines (Klohs et al. (1972) Arzneimittelforschung 22:132-3). Parmi ses avantages figurent notamment l'absence d'effets dépresseurs respiratoires. Son mode d'action est encore mal connu mais semble faire appel à une inhibition de la recapture des monoamines, ce qui le distingue du paracétamol et des anti-inflammatoires non stéroïdiens (AINS). Les AINS sont des inhibiteurs plus ou moins sélectifs de l'isoforme 2 de la cyclooxygénase (COX-2) qui ont ainsi pour effet de réduire la synthèse de prostaglandines dans les systèmes nerveux centraux et périphériques.

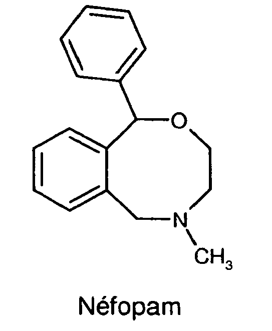

A l'heure actuelle, le néfopam est principalement utilisé pour le traitement de la douleur post-opératoire. En France, on le trouve ainsi administré chez environ 20% des patients ayant subi une intervention chirurgicale (Fletcher et al. (2008) Pain 137:441-51). Toutefois, son efficacité analgésique propre n'est parfois pas suffisante dans les chirurgies douloureuses, et, de fait, il est fréquemment associé à d'autres analgésiques, notamment la morphine, le paracétamol et le kétoprofène (AINS) (Fletcher et al. (2008) Pain 137:441-51). Ces associations souffrent cependant de certaines limitations.

Ainsi, bien qu'elle permette une réduction notable de la quantité de morphine administrée pour une même efficacité analgésique, l'association néfopam-morphine ne permet pas toujours de diminuer les doses de morphine de manière suffisante pour supprimer les manifestations indésirables liées à son utilisation (Du Manoir et al. (2003) Br. J. Anaesth. 91:836-841). L'effet de l'association néfopam-kétoprofène a, quant à elle, été évaluée en post-opératoire comme étant synergique, mais seulement après des chirurgies modérément douloureuses (Delage et al. (2005) Anesthesiology 102:12111216). En outre, l'utilisation de kétoprofène est souvent limitée dans le temps en raison de sa mauvaise tolérance digestive.

II reste donc à trouver une association permettant de profiter des avantages du néfopam tout en offrant un certain confort d'utilisation pour le patient.

A ce titre, l'efficacité analgésique d'une association du néfopam avec le diclofénac, un autre AINS, a également été évaluée sans qu'aucun bénéfice ne puisse être mis en évidence par rapport à l'utilisation de diclofénac seul (Moffat et al. (1990) Anesthesia 45:302-305).

L'ibuprofène est également un AINS qui entraîne une analgésie efficace, bien qu'inférieure à celle du kétoprofène (Mills et al. (1973) British Medical Journal 4:82-84). A l'heure actuelle l'ibuprofène n'est quasiment pas utilisé dans la prise en charge de la douleur post-opératoire (Fletcher et al. (2008) Pain 137:441-51).

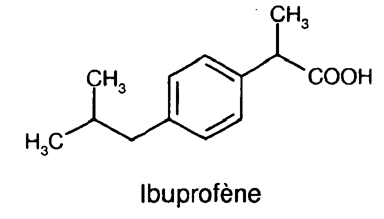

### Résumé de l'invention

La présente invention découle de la découverte inattendue, par les inventeurs, d'un effet analgésique synergique entre le néfopam et l'ibuprofène dans un modèle animal de douleur aiguë.

Ainsi, la présente invention concerne une composition pharmaceutique comprenant à titre de substances actives :
a) au moins un composé de formule générale (I) suivante : 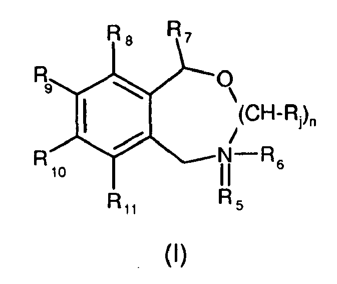 dans laquelle :
   - R₅ représente O ou aucun groupement ;
   - R₆ représente H ou un groupement alkyle contenant de 1 à 6 atomes de carbone ;
   - n représente un nombre entier de 2 à 4 ;
   - j représente un nombre entier variant de 1 à n ;
   - Rⱼ, identique ou différent pour chaque carbone substitué, représente H ou un groupement alkyle contenant de 1 à 6 atomes de carbone ;
   - R₇ représente un groupement phényle éventuellement substitué par un ou plusieurs groupements, identiques ou différents, choisi parmi la liste comprenant H, un groupement alkyle contenant de 1 à 6 atomes de carbone, un groupement alkoxy contenant de 1 à 6 atomes de carbones, un groupement trifluorométhyle,
   ou un atome d'halogène ;
   - R₈, R₉, R₁₀, R₁₁ identiques ou différents représentent H, un groupement alkyle contenant de 1 à 6 atomes de carbone, un groupement alkoxy contenant de 1 à 6 atomes de carbone, un groupement trifluorométhyle, ou un atome d'halogène ;
   ou un sel pharmaceutiquement acceptable de ce composé ; et
b) au moins un composé de formule générale (V) suivante : 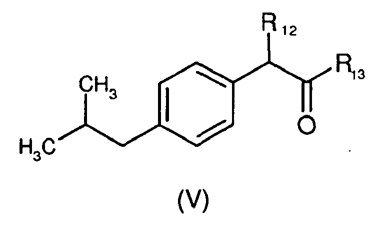
dans laquelle :
- R₁₂ représente H ou un groupement alkyle contenant de 1 à 6 atomes de carbone ;
- R₁₃ représente un groupement OR₁₄ ou NR₁₅R₁₆ ;
- R₁₄ représente H, un groupement alkyle contenant de 1 à 6 atomes de carbone, un groupement aryle contenant de 6 à 10 atomes de carbone, ou un groupement aralkyle ou alkaryle contenant de 7 à 20 atomes de carbone ;
- R₁₅ et R₁₆ identiques ou différents représentent H, OH, un groupement alkyle contenant de 1 à 6 atomes de carbone, un groupement aryle contenant de 6 à 10 atomes de carbone, ou un groupement aralkyle ou alkaryle contenant de 7 à 20 atomes de carbone ;
ou un sel pharmaceutiquement acceptable de composé ;
éventuellement associés à un ou plusieurs excipients pharmaceutiquement acceptables,
notamment pour induire une analgésie ou pour son utilisation dans la prévention
ou le traitement de la douleur.

La présente invention concerne également un composé de formule générale (I) telle que définie ci-dessus, ou un sel pharmaceutiquement acceptable de celui-ci, en combinaison avec un composé de formule générale (V) telle que définie ci-dessus, le composé de formule (V) étant représenté par une des formules (VII), (VIII) et (IX) montrées ci-bas, ou un sel pharmaceutiquement acceptable de celui-ci, pour leur utilisation à titre de médicament, en particulier pour induire une analgésie ou pour prévenir ou traiter la douleur.

La présente invention concerne également l'utilisation d'un composé de formule générale (I) telle que définie ci-dessus, ou d'un sel pharmaceutiquement acceptable de celui-ci, en combinaison avec un composé de formule générale (V) telle que définie ci-dessus, le composé de formule (V) étant représenté par une des formules (VII), (VIII) et (IX) montrées ci-bas, ou d'un sel pharmaceutiquement acceptable de celui-ci, pour la préparation d'un médicament analgésique ou destiné à la prévention ou au traitement de la douleur.

La présente invention concerne également une méthode d'induction d'une analgésie ou de prévention ou traitement de la douleur chez un individu, dans laquelle on administre à l'individu une quantité prophylactiquement ou thérapeutiquement efficace d'au moins un composé de formule générale (I) telle que définie ci-dessus, ou d'un sel pharmaceutiquement acceptable de celui-ci, et une quantité prophylactiquement ou thérapeutiquement efficace et d'au moins un composé de formule générale (V) telle que définie ci-dessus, le composé de formule (V) étant représenté par une des formules (VII), (VIII) et (IX), ou d'un sel pharmaceutiquement acceptable de celui-ci.

La présente invention concerne également des produits contenant :
- au moins un composé de formule générale (I) telle que définie ci-dessus, ou un sel pharmaceutiquement acceptable de celui-ci, et
- au moins un composé de formule générale (V) telle que définie ci-dessus, le composé de formule (V) étant représenté par une des formules (VII), (VIII) et (IX), ou un sel pharmaceutiquement acceptable de celui-ci,
comme produit de combinaison pour une utilisation ensemble ou de manière séparée pour induire une analgésie ou pour prévenir ou traiter la douleur.

Dans un mode de réalisation préféré de la composition pharmaceutique, des composés, de l'utilisation, de la méthode et des produits définis ci-dessus, au moins un composé analgésique ou antalgique additionnel, différent des composés de formules générales (I) et (V) telles que définies ci-dessus, le composé de formule (V) étant représenté par une des formules (VII), (VIII) et (IX), ou de leurs sels pharmaceutiquement acceptables, est ajouté en combinaison avec les composés de formules générales (I) et (V) telles que définies ci-dessus. Le composé de formule (V) étant représenté par une des formules (VII), (VIII) et (IX).

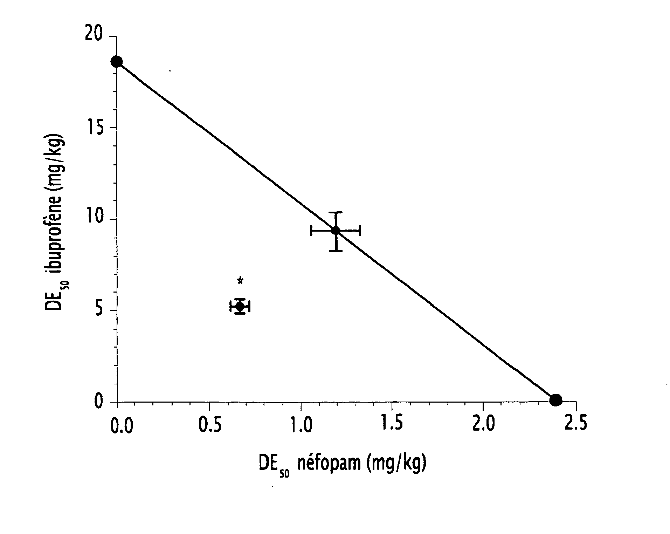

### Description de la figure 1

La figure 1 représente l'isobologramme de l'association ibuprofène-néfopam dans le test des crampes induites par l'acide acétique chez la souris. Le symbole étoile (*) indique que le point représentatif de la DE50 de l'association ibuprofène-néfopam mesurée expérimentalement est situé sous la droite d'additivité de manière statistiquement significative.

### Description détaillée de l'invention

### Prévention ou traitement de la douleur

L'expression « traiter la douleur » signifie diminuer ou abolir une douleur ou la sensibilité à cette douleur. L'expression « prévenir la douleur » signifie que les composés de formules générales (I) et (V) telles définies ci-dessus, le composé de formule (V) étant représenté par une des formules (VII), (VIII) et (IX), ou des sels pharmaceutiquement acceptables de ceux-ci, sont administrés à un individu avant que cet individu ne perçoive la douleur à traiter.

L'expression « induire une analgésie » signifie aussi bien diminuer ou abolir une douleur que la sensibilité à cette douleur. On considère ici que cette expression est équivalente à « une utilisation en tant qu'analgésique ou antalgique ».

De préférence, la douleur prévenue ou traitée selon l'invention est une douleur aiguë. L'expression « douleur aiguë » est bien connue de l'homme du métier. Elle s'oppose à la notion de « douleur chronique ». D'une manière générale on considère qu'une douleur aiguë est une douleur dont la durée est inférieure à 3 mois.

De préférence également, la douleur prévenue ou traitée selon l'invention est une douleur aiguë post-opératoire. On désigne par « douleur aiguë post-opératoire » une douleur qui trouve son origine dans une intervention chirurgicale. En particulier, une intervention chirurgicale lors de laquelle est réalisée une incision, dont la cicatrisation crée une douleur de type inflammatoire avec une participation hyperalgésique, pendant une durée moyenne de 5 à 7 jours (Chauvin et Clergue (1998) Ann. Fr. Anesth. Réanim. 17:444).

De préférence, l'intensité de la douleur prévenue ou traitée selon l'invention est au moins modérée, plus préférablement au moins sévère (également nommée forte). Les notions de « douleur modérée » ou de « douleur sévère » sont bien connues de l'homme du métier. A titre d'exemple, on considère généralement qu'une douleur modérée correspond à un index de 4 à 6 et une douleur sévère à un index de 7 à 9 sur une échelle numérique de douleur graduée de 0 à 10. Sur cette même échelle l'index 0 correspond à une absence de douleur, l'index 1 à 3 à une douleur légère, et l'index 10 à la douleur maximale imaginable.

### Composé de formule général (I)

De préférence, le composé de formule générale (I) définie ci-dessus est représenté par l'une des formules (II), (III) et (IV) suivantes :

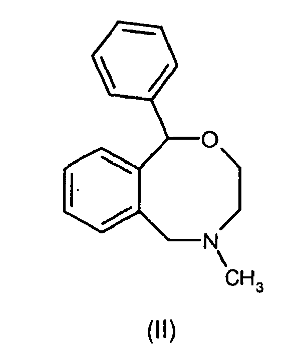

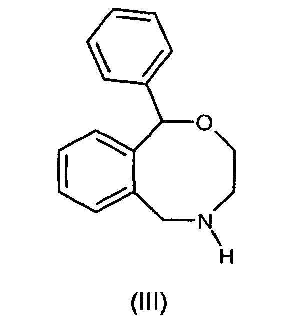

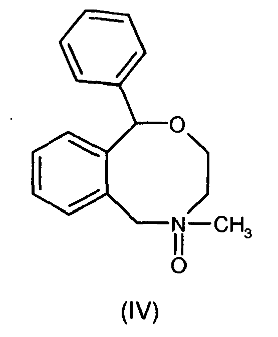

La formule (II) représente le néfopam, et les formules (III) et (IV) représentent respectivement deux métabolites du néfopam à savoir le desméthyle néfopam et le *N*-oxyde néfopam.

De manière particulièrement préférée, le composé de formule générale (I) telle que définie ci-dessus est représenté par la formule (II) ci-dessus.

Comme on l'entend ici, la formule générale (I) définie ci-dessus représente également les stéréoisomères et mélanges de stéréoisomères, notamment le mélange racémique, des composés de formule (I).

Les sels pharmaceutiquement acceptables des composés de formule générale (I) définie ci-dessus apparaîtront clairement à l'homme du métier. En particulier, les sels de chlorhydrate des composés de formule générale (I) telle que définie ci-dessus sont préférés.

Le chlorhydrate de néfopam est le composé de formule générale (I) telle que définie ci-dessus le plus préféré pour la mise en oeuvre de l'invention.

### Composé de formule générale (V)

Le composé de formule générale (V) définie ci-dessus est représenté par les formules (VII), (VIII) et (IX) suivantes :

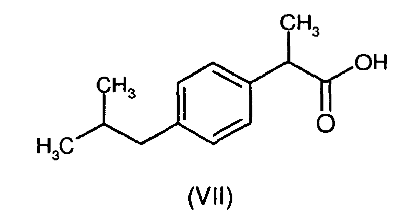

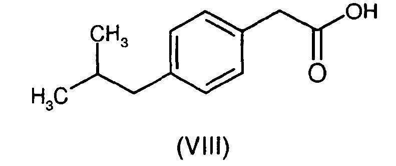

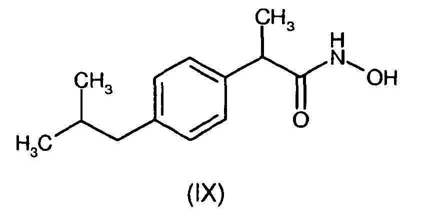

Les formules (VII), (VIII) et (IX) ci-dessus représentent respectivement l'ibuprofène, l'ibufénac et l'ibuproxam.

De manière davantage préférée le composé de formule générale (V) définie ci-dessus est représenté par la formule (VII) ci-dessus et de manière particulièrement préférée par la formule (X) suivante :

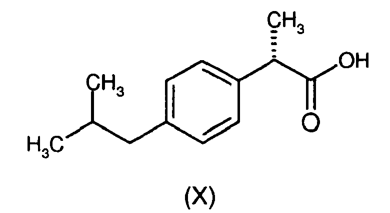

La formule (X) représente la forme S de l'ibuprofène qui porte l'essentiel des propriétés analgésiques de l'ibuprofène.

Comme on l'entend ici, les formules (V), (VII) et (IX) définies ci-dessus représentent également les stéréoisomères et mélanges de stéréoisomères, notamment le mélange racémique, des composés de formules (V), (VII) et (IX).

Les sels pharmaceutiquement acceptables des composés de formule générale (V) définie ci-dessus apparaîtront clairement à l'homme du métier. En particulier, les sels de lysine, notamment de L-lysine, des composés de formule générale (V) telle que définie ci-dessus sont préférés, tels que le sel de lysine de l'ibuprofène ou le sel monohydraté de L-lysine de la forme S de l'ibuprofène.

Par ailleurs, comme cela apparaîtra clairement à l'homme du métier, il est aisé de synthétiser des prodrogues des composés de formules, (VII) et (VIII) définies ci-dessus, c'est-à-dire des composés qui sont rapidement transformés *in vivo* pour donner le composé de formules, (VII) et (VIII) définies ci-dessus, par exemple par hydrolyse dans le sang. Aussi, des prodrogues des composés de formules, (VII) et (VIII) définies ci-dessus qui sont représentées par les composés de formule (V) pour lesquelles R₁₃ est différent de OH, tel que l'ibuproxam, sont également prévues pour la mise en oeuvre selon l'invention.

### Administration

Comme on l'entend ici l'expression « en combinaison » ou « produit de combinaison » signifie que le composé de formule générale (I) telle que définie ci-dessus, ou un sel pharmaceutiquement acceptable de celui-ci, et le composé de formule générale (V) telle que définie ci-dessus, le composé de formule (V) étant représenté par une des formules (VII), (VIII) et (IX), ou un sel pharmaceutiquement acceptable de celui-ci, peuvent être associés au sein d'une même composition pharmaceutique, et donc être administrés ensemble, ou bien être administrés de manière séparée, c'est-à-dire selon des voies d'administration distinctes et/ou des régimes d'administration distincts, sous réserve que lorsqu'ils sont administrés de manière séparée les périodes d'activité analgésique respectives de chacun des deux composés se recouvrent en totalité ou en partie, notamment de manière que les composés puissent coopérer pour exercer un effet analgésique synergique.

Ainsi, lorsque les composés sont administrés de manière séparée, le composé de formule générale (I) telle que définie ci-dessus, ou son sel pharmaceutiquement acceptable, sera de préférence administré dans les 24 heures suivant l'administration du composé de formule générale (V) telle que définie ci-dessus, le composé de formule (V) étant représenté par une des formules (VII), (VIII) et (IX), ou de son sel pharmaceutiquement acceptable, et son administration sera éventuellement poursuivie les jours suivants. Réciproquement, le composé de formule générale (V) telle que définie ci-dessus, le composé de formule (V) étant représenté par une des formules (VII), (VIII) et (IX), ou son sel pharmaceutiquement acceptable, sera de préférence administré dans les 24 heures suivant l'administration du composé de formule générale (I) telle que définie ci-dessus, ou de son sel pharmaceutiquement acceptable, et son administration sera éventuellement poursuivie les jours suivants.

De préférence, le composé de formule générale (I) telle que définie ci-dessus, ou son sel pharmaceutiquement acceptable, est administré ou se trouve sous une forme convenant à une administration par voie orale, intraveineuse ou intramusculaire.

De préférence, le composé de formule générale (V) telle que définie ci-dessus, le composé de formule (V) étant représenté par une des formules (VII), (VIII) et (IX), ou son sel pharmaceutiquement acceptable, est administré ou se trouve sous une forme convenant à une administration par voie orale, injectable ou locale.

De préférence, lorsque le composé de formule générale (I) telle que définie ci-dessus, ou son sel pharmaceutiquement acceptable, et le composé de formule générale (V) telle que définie ci-dessus, le composé de formule (V) étant représenté par une des formules (VII), (VIII) et (IX), ou son sel pharmaceutiquement acceptable, sont associés au sein d'une même composition pharmaceutique, celle-ci est administrée ou se trouve sous une forme convenant à une administration par voie orale, injectable ou locale.

De préférence, le composé de formule (I) telle que définie ci-dessus, ou son sel pharmaceutiquement acceptable, notamment le chlorhydrate de néfopam, se trouve contenu dans les compositions pharmaceutiques ou les produits définis ci-dessus, ou est administré, à une dose unitaire de 1 à 120 mg, plus préférablement à une dose unitaire de 20 mg.

De préférence, le composé de formule (V) telle que définie ci-dessus, le composé de formule (V) étant représenté par une des formules (VII), (VIII) et (IX), ou son sel pharmaceutiquement acceptable, notamment l'ibuprofène, en particulier sous sa forme S, se trouve contenu dans les compositions pharmaceutiques ou les produits définis ci-dessus, ou est administré, à une dose unitaire de 1 à 2400 mg, plus préférablement à une dose unitaire de 400 mg.

### Composé analgésique ou antalgique additionnel

N'importe quel composé analgésique ou antalgique peut convenir à titre de composé analgésique ou antalgique additionnel selon l'invention, il est préféré, toutefois, qu'il s'agisse :
- d'un morphinique, tel que la morphine, le fentanyl, le remifentanil, l'alfentanil, le sufentanyl, la nalbuphine, la pentazocine, la codéine, l'hydrocodéine, le dextropropoxyphène, le tramadol, la buprénorphine, l'hydromorphone, l'oxycodone, ou la péthidine ;
- d'un anti-inflammatoire non stéroïdien (AINS), tel que le kétoprofène, l'acide acétylsalicylique, l'acide méfénamique, le fénoprofène, l'acéclofénac, l'acide tiaprofénique, l'alminoprofène, le diclofénac, l'étodolac, le flurbiprofène, la nabumétone, le naproxène, le méloxicam, le piroxicam, le ténoxicam, l'indométacine, le sulindac, le célécoxib, le parécoxib, la floctafénine, la phénylbutazone, ou le nimésulide (liste non exhaustive) ;
- du paracétamol, du ziconitide, ou de la caféine

### Exemple

L'effet analgésique d'une association néfopam - ibuprofène a été étudié dans le modèle de crampes abdominales induites chez la souris par l'administration intrapéritonéale d'acide acétique. Ce modèle de douleur induite par une substance chimique correspond à une douleur inflammatoire, viscérale et aiguë.

### A. Matériel et méthodes

### 1. Animaux

Des souris mâles CD1 (élevage C. River) de poids compris entre 25 et 30 grammes sont utilisées après une acclimatation d'au moins 7 jours dans l'animalerie (t° = 22 ± 2 ; hygrométrie = 50 ± 20 % ; nourriture SAFE "A04"; cycle nycthéméral : 12 h/12 h (lumière : 7 h/19 h - obscurité : 19 h/7 h)).

### 2. Protocole

Le jour de l'expérimentation, les souris non à jeun sont pesées, marquées et réparties au hasard par lot de 10. La solution d'acide acétique (Sigma) est préparée à 0,6 % (0,1 ml/10 g) soit 60 mg d'acide acétique dans 10 ml de NaCl à 0,9 %.

A t = 0, la souris reçoit le produit étudié ou le liquide véhicule par voie sous cutanée (0,1 ml/10 g). A t = 30 minutes, l'acide acétique est injecté par voie intrapéritonéale (0,1 ml/10 g).

On compte le nombre de crampes abdominales de 5 à 20 minutes après l'injection d'acide acétique. On considère comme positives les crampes abdominales franches caractérisées par l'étirement des pattes postérieures et/ou le creusement des flancs avec une torsion.

### 3. Produits

Le chlorhydrate de néfopam (Biocodex, lot 38) (ci-après désigné simplement néfopam) est solubilisé dans l'eau distillée ou du NaCl à 0,9 %. L'ibuprofène (Sigma, réf I-4883) est mis en suspension dans du Tween 80 à 1 %.

### 4. Analyse statistique

Le test utilisé est une analyse de variance à partir de 3 groupes et un test t de Student pour 2 groupes. Ensuite, on détermine le ou les groupes traités qui diffèrent du groupe témoin.

Le calcul des doses efficaces à 50 % d'effet antinociceptif (DE50) se fait à l'aide du programme PharmToolsPro (version 1.1.27, McCary Group Inc.) selon la méthode de Tallarida (2000) Drugs synergism and dose-effect data analysis CRC Press). Au moins 10 souris sont utilisées pour chaque dose, et au moins 3 doses sont employées pour déterminer la courbe dose-réponse. La dose qui produit 50 % d'effet antinociceptif (diminution de 50 % du nombre de crampes) est calculée par une analyse de régression linéaire standard de la courbe dose-réponse.

L'interaction est évaluée par une analyse isobolographique de la co-administration d'une combinaison de doses à rapport fixe selon Tallarida (2000) *op. cit*., Tallarida et al. (1989) Life Sci. 45:947-961 et Tallarida et al. (1997) Life Sci. 61:PL417-PL425. L'isobologramme est construit en joignant la DE50 de l'ibuprofène avec celle du néfopam pour obtenir la ligne d'additivité. La DE50 de l'association est déterminée par analyse de régression linéaire de la courbe dose-réponse, et elle est comparée par un test *t* à une DE50 additive théorique obtenue à l'aide du logiciel PharmToolsPro.

### B. Résultats

### 1. Néfopam seul

L'administration sous-cutanée de néfopam entraîne une inhibition dose-dépendante du nombre de crampes induites par l'acide acétique chez la souris (**Tableau 1**). La DE50 mesurée du néfopam est de 2,395 ± 0,215 mg/kg.

### 2. Ibuprofène seul

L'ibuprofène administré par voie sous-cutanée diminue de façon dose-dépendante le nombre de crampes induites par l'acide acétique chez la souris, avec une DE50 de 18,581 ± 2,398 mg/kg (**Tableau 1**).

Cette valeur de DE50 est à rapprocher des valeurs obtenues dans le même modèle avec une administration par voie orale. Dans ce cadre, l'ibuprofène a ainsi montré une activité antinociceptive significative à partir de 30 mg/kg (Dolezal & Ksiak (2002) Physiol Res. 51:179-184) ou avec une DE50 de 95 mg/kg (Jones et al. (2005) J. Pharmacol. Exp. Ther. 312:726-732).

**Tableau 1 : effets propres du néfopam et de l'ibuprofène administrés seuls**

| **Produits** | **n** | **Nombre de crampes** | **% variation** | **ANOVA** |
|---|---|---|---|---|
| **(mg/kg)** | | **(moyennes ± esm)** | | |
| **Néfopam** | | | | |
| 0 | 18 | 37,1 ± 2,8 | | |
| 0,3 | 9 | 33,6 ± 3,8 | -9 | ns |
| 1,0 | 10 | 28,0 ± 6,3 | -25 | ns |
| 3,0 | 18 | 16,7 ± 2,0 | -55 | p < 0,05 |
| 10,0 | 10 | 7,1 ± 1,7 | -81 | p < 0,05 |
| 20,0 | 10 | 0,6 ± 0,3 | -98 | p < 0,05 |
| | | **DE50 (mg/kg) 2,395 ± 0,215** | | |

| **Ibuprofène** | | | | |
|---|---|---|---|---|
| 0 | 18 | 37,6 ± 1,5 | | |
| 1 | 10 | 40,2 ± 3,2 | +7 | Ns |
| 3 | 10 | 43,0 ± 6,6 | +14 | Ns |
| 10 | 10 | 26,8 ± 3,9 | -29 | Ns |
| 20 | 9 | 22,0 ± 2,0 | -41 | p < 0,05 |
| 30 | 9 | 12,1 ± 2,7 | -68 | p < 0,05 |
| 50 | 9 | 5,9 ± 1,3 | -84 | p < 0,05 |
| 100 | 9 | 6,0 ± 1,4 | -84 | p < 0,05 |
| | | **DE50 (mg/kg) 18,581 ± 2,398** | | |

| | | | | |
|---|---|---|---|---|
| (p < 0,05 : test statistique ANOVA suivi Bonferoni ou Dunn) | | | | |

### 3. Association néfopam - ibuprofène

Dans un premier temps, on détermine, à l'aide du logiciel PharmToolsPro, la proportion fixe de chaque produit pour un niveau d'efficacité de 50 % et la DE50 théorique qui se situe sur la droite d'additivité, selon Tallarida (2000) *op. ci*t. On obtient ainsi une proportion de 0,114 pour le néfopam et de 0,886 pour l'ibuprofène et une DE50 théorique de 10,495 ± 1,205 mg/kg.

Dans un deuxième temps, des compositions ayant une proportion de 11,4 % de néfopam et de 88,6 % d'ibuprofène sont étudiées dans le modèle animal afin d'obtenir une DE50 expérimentale qui sera comparée à la DE50 théorique de la droite d'additivité. Le **Tableau 2** montre les résultats expérimentaux obtenus. La DE50 expérimentale est de 5,870 ± 0,425 mg/kg (correspondant 0,669 ± 0,048 mg/kg de néfopam et 5,201 ± 0,277 mg/kg d'ibuprofène).

Enfin, dans un dernier temps, la DE50 expérimentale est placée sur l'isobologramme obtenu à partir des données du **Tableau 1** (**Figure 1**). On observe que la DE50 expérimentale de l'association néfopam - ibuprofène est située en dessous de la droite d'additivité, où se situe la DE50 théorique correspondant à une additivité simple. L'interaction entre le néfopam et l'ibuprofène se situe donc dans la zone de supra-additivité indiquant une relation synergique entre les deux composés. Par ailleurs, l'analyse statistique donne un *t_{expérimental}* de 4,276 qui est supérieur au *T_{théorique}* de 2,381, par conséquent la différence entre la DE50 expérimentale et la DE50 théorique est significative.

**Tableau 2 : effet de la co-administration du néfopam et de l'ibuprofène**

| **Néfopam** | **Ibuprofène** | **n** | **Nombre de crampes** | **% variation** | **ANOVA** |
|---|---|---|---|---|---|
| **(mg/kg)** | **(mg/kg)** | | **(moyennes esm)** | | |
| 0 | 0 | 9 | 27,9 ± 3,1 | | |
| 0,075 | 0,58 | 10 | 27,7 ± 4,1 | -1 | ns |
| 0,15 | 1,16 | 10 | 22,4 ± 4,2 | -20 | ns |
| 0,30 | 2,32 | 10 | 18,1 ± 3,6 | -35 | ns |
| 0,60 | 4,65 | 8 | 16,6 ± 3,1 | -41 | ns |
| 1,20 | 9,30 | 9 | 9,2 ± 2,3 | -67 | p < 0,05 |
| 2,40 | 18,60 | 10 | 7,0 ± 1,5 | -75 | p < 0,05 |
| 4,80 | 37,20 | 9 | 1,7 ± 1,0 | -94 | p < 0,05 |
| | | | **DE50 (mg/kg) 5,870 ± 0,425** | | |

| | | | | | |
|---|---|---|---|---|---|
| (p < 0,05 : test statistique ANOVA suivi Bonferoni ou Dunn) | | | | | |

## Revendications

1. Composition pharmaceutique comprenant à titre de substances actives :
a) au moins un composé de formule générale (I) suivante : 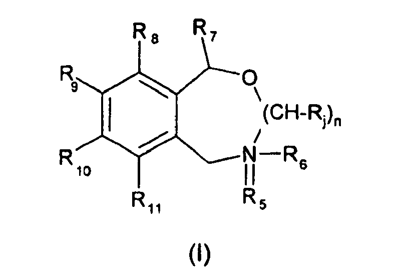 dans laquelle :
- R₅ représente O ou aucun groupement ;
- R₆ représente H ou un groupement alkyle contenant de 1 à 6 atomes de carbone ;
- n représente un nombre entier de 2 à 4 ;
- j représente un nombre entier variant de 1 à n ;
- Rⱼ, identique ou différent pour chaque carbone substitué, représente H ou un groupement alkyle contenant de 1 à 6 atomes de carbone ;
- R₇ représente un groupement phényle éventuellement substitué par un ou plusieurs groupements, identiques ou différents, choisi parmi la liste comprenant H, un groupement alkyle contenant de 1 à 6 atomes de carbone, un groupement alkoxy contenant de 1 à 6 atomes de carbones, un groupement trifluorométhyle, ou un atome d'halogène ;
- R₈, R₉, R₁₀, R₁₁ identiques ou différents représentent H, un groupement alkyle contenant de 1 à 6 atomes de carbone, un groupement alkoxy contenant de 1 à 6 atomes de carbone, un groupement trifluorométhyle, ou un atome d'halogène ;
ou un sel pharmaceutiquement acceptable de ce composé ; et
b) au moins un composé d'une des formules (VII), (VIII), et (IX) suivantes : 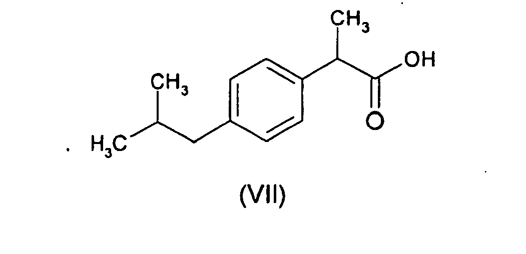 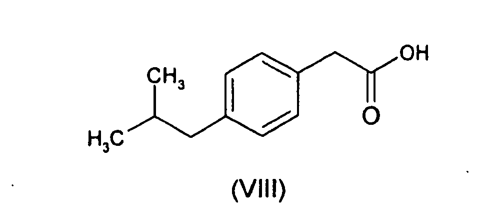 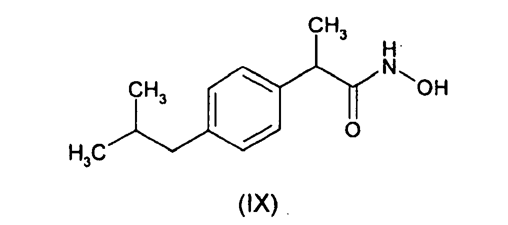 ou un sel pharmaceutiquement acceptable de composé ;
éventuellement associés à une ou plusieurs excipients pharmaceutiquement acceptables.

2. Composition pharmaceutique selon la revendication 1, dans laquelle le composé de formule (I) est représenté par une des formules (II), (III) et (IV) suivantes :

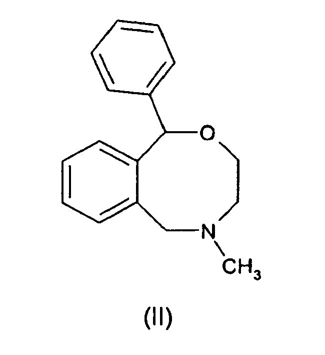

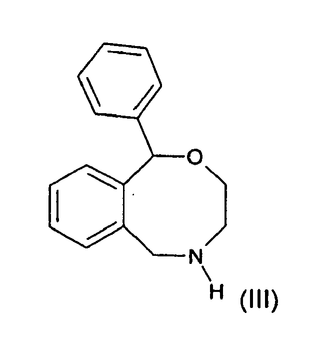

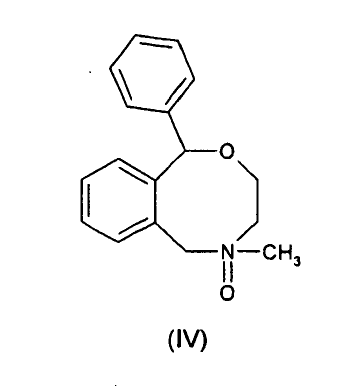

3. Composition pharmaceutique selon la revendication 1 ou 2, dans laquelle le composé de formule (VII) est représenté par la formule (X) suivante :

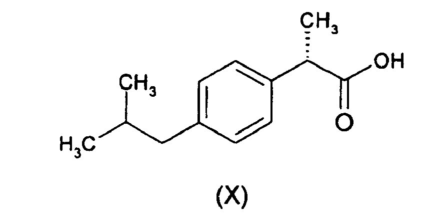

4. Composition pharmaceutique selon l'une des revendications 1 à 3, comprenant :
a) du chlorhydrate de néfopam, et
b) de l'ibuprofène.

5. Composition pharmaceutique selon l'une des revendications 1 à 4, comprenant une dose unitaire de 1 à 120 mg du composé de formule (I) ou d'un sel pharmaceutiquement acceptable de celui-ci.

6. Composition pharmaceutique selon l'une des revendications 1 à 5, comprenant une dose unitaire de 1 à 2400 mg du composé de formule (VII), (VIII) ou (IX), ou d'un sel pharmaceutiquement acceptable de celui-ci.

7. Composition pharmaceutique selon l'une des revendications 1 à 6, convenant pour une administration par voie orale, injectable ou locale.

8. Composition pharmaceutique selon l'une des revendications 1 à 7, pour induire une analgésie ou pour son utilisation dans la prévention ou le traitement de la douleur.

9. Composé de formule générale (I), ou sel pharmaceutiquement acceptable de celui-ci, tel que défini dans l'une des revendications 1, 2 et 4, en combinaison avec le composé de formule générale (VII), (VIII) ou (IX), ou un sel pharmaceutiquement acceptable de celui-ci, tel que défini dans l'une des revendications 1, 3 et 4, pour leur utilisation à titre de médicament.

10. Composés ou sels pharmaceutiquement acceptables de ceux-ci selon la revendication 9, dans lesquels le composé de formule (I), ou un sel pharmaceutiquement acceptable de celui-ci, est administré à la dose unitaire de 1 à 120 mg.

11. Composés ou sels pharmaceutiquement acceptables de ceux-ci selon la revendication 9 ou 10, dans lesquels le composé de formule (VII), (VIII) ou (IX), ou un sel pharmaceutiquement acceptable de celui-ci, est administré à la dose unitaire de 1 à 2400 mg.

12. Composés ou sels pharmaceutiquement acceptables de ceux-ci selon l'une des revendications 9 à 11, dans lesquels le composé de formule (I), ou un sel pharmaceutiquement acceptable de celui-ci, est administré par voie intraveineuse, orale, ou intramusculaire.

13. Composés ou sels pharmaceutiquement acceptables de ceux-ci selon l'une des revendications 9 à 12, dans lesquels le composé de formule (VII), (VIII) ou (IX), ou un sel pharmaceutiquement acceptable de celui-ci, est administré par voie orale, injectable ou locale.

14. Composés ou sels pharmaceutiquement acceptables de ceux-ci selon l'une des revendications 9 à 13, pour induire une analgésie ou pour leur utilisation dans la prévention ou le traitement de la douleur.

15. Produits contenant :
- au moins un composé de formule générale (I), ou un sel pharmaceutiquement acceptable de celui-ci, tel que défini dans l'une des revendications 1, 2 et 4, et
- au moins un composé de formule (VII), (VIII) ou (IX), ou un sel pharmaceutiquement acceptable de celui-ci, tel que défini dans l'une des revendications 1, 3 et 4,
comme produit de combinaison pour une utilisation ensemble ou de manière séparée pour induire une analgésie ou pour prévenir ou traiter la douleur.

16. Produits selon la revendication 15, comprenant une dose unitaire de 1 à 120 mg du composé de formule (I), ou d'un sel pharmaceutiquement acceptable de celui-ci.

17. Produits selon la revendication 15 ou 16, comprenant une dose unitaire de 1 à 2400 mg du composé de formule (VII), (VIII) ou (IX), ou d'un sel pharmaceutiquement acceptable de celui-ci.

18. Produits selon l'une des revendications 15 à 17, dans lequel le compose de formule (I), ou un sel pharmaceutiquement acceptable de celui-ci, est sous une forme administrable par voie intraveineuse, orale, ou intramusculaire.

19. Produits selon l'une des revendications 15 à 18, dans lesquels le composé de formule (VII), (VIII) ou (IX), ou un sel pharmaceutiquement acceptable de celui-ci, est sous une forme administrable par voie orale, injectable ou locale.

## Patentansprüche

1. Pharmazeutische Zusammensetzung, umfassend als aktive Substanzen:
a) wenigstens eine Verbindung der folgenden allgemeinen Formel (I): 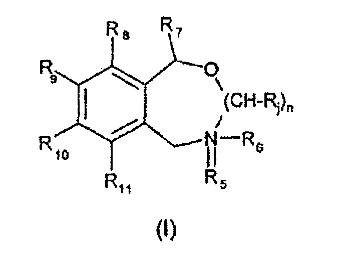 in der:
- R⁵ O oder keine Gruppierung darstellt;
- R⁶ H oder eine Alkylgruppierung, die 1 bis 6 Kohlenstoffatome enthält, darstellt;
- n eine ganze Zahl von 2 bis 4 darstellt;
- j eine ganze Zahl von 1 bis n darstellt;
- Rⱼ, gleich oder unterschiedlich für jeden substituierten Kohlenstoff, H oder eine Alkylgruppierung, die 1 bis 6 Kohlenstoffatome enthält, darstellt;
- R⁷ eine Phenylgruppierung darstellt, die gegebenenfalls mit einer oder mehreren Gruppierung(en) substituiert ist, die gleich oder unterschiedlich sind, die ausgewählt ist (sind) aus der Liste, die H, eine Alkylgruppierung, die 1 bis 6 Kohlenstoffatome enthält, eine Alkoxygruppierung, die 1 bis 6 Kohlenstoffatome enthält, eine Trifluormethylgruppierung oder ein Halogenatom umfasst;
- R⁸, R⁹, R¹⁰, R¹¹, die gleich oder unterschiedlich sind, H, eine Alkylgruppierung, die 1 bis 6 Kohlenstoffatome enthält, eine Alkoxygruppierung, die 1 bis 6 Kohlenstoffatome enthält, eine Trifluormethylgruppierung oder ein Halogenatom darstellen;
oder ein pharmazeutisch annehmbares Salz dieser Verbindung und
b) wenigstens eine Verbindung einer der folgenden Formeln (VII), (VIII) und (IX) : 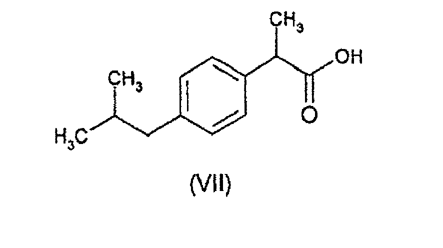 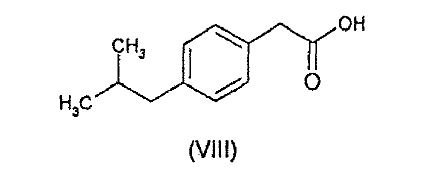 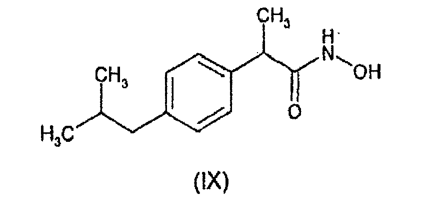 oder ein pharmazeutisch annehmbares Salz dieser Verbindung;
- gegebenenfalls kombiniert mit einem pharmazeutisch annehmbaren Exzipiens oder mehreren pharmazeutisch annehmbaren Exzipientien.

2. Pharmazeutische Zusammensetzung gemäß Anspruch 1, in der die Verbindung der Formel (I) durch eine der folgenden Formeln (II), (III) und (IV) dargestellt wird:

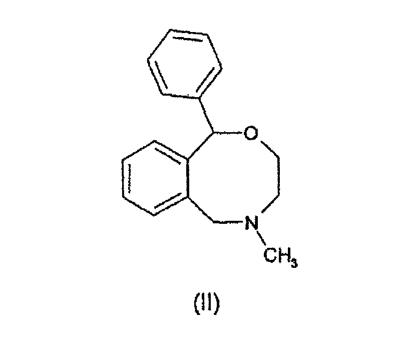

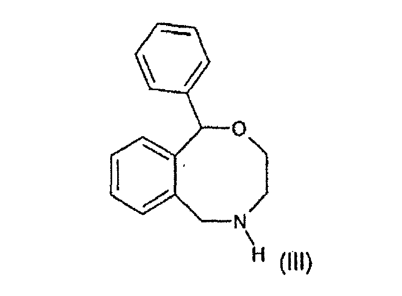

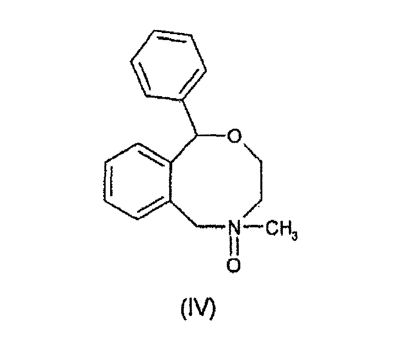

3. Pharmazeutische Zusammensetzung gemäß Anspruch 1 oder 2, in der die Verbindung der Formel (VII) durch die folgende Formel (X) dargestellt wird:

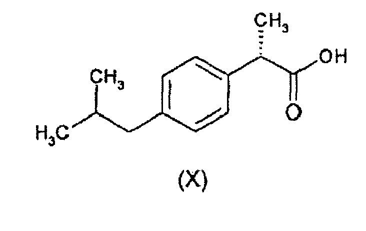

4. Pharmazeutische Zusammensetzung gemäß einem der Ansprüche 1 bis 3, umfassend:
a) Nefopam-Hydrochlorid und
b) Ibuprofen.

5. Pharmazeutische Zusammensetzung gemäß einem der Ansprüche 1 bis 4, umfassend eine Einheitsdosis von 1 bis 120 mg der Verbindung der Formel (I) oder eines pharmazeutisch annehmbaren Salzes dieser.

6. Pharmazeutische Zusammensetzung gemäß einem der Ansprüche 1 bis 5, umfassend eine Einheitsdosis von 1 bis 240 mg der Verbindung der Formel (VII), (VIII) oder (IX) oder eines pharmazeutisch annehmbaren Salzes dieser.

7. Pharmazeutische Zusammensetzung gemäß einem der Ansprüche 1 bis 6, die für eine Verabreichung auf oralem, Injektions- oder lokalem Weg angepasst ist.

8. Pharmazeutische Zusammensetzung gemäß einem der Ansprüche 1 bis 7, um eine Analgesie zu induzieren oder für ihre Verwendung in der Prävention oder der Behandlung von Schmerz.

9. Verbindung der allgemeinen Formel (I) oder pharmazeutisch annehmbares Salz dieser, wie in einem der Ansprüche 1, 2 und 4 definiert, in Kombination mit der Verbindung der allgemeinen Formel (VII), (VIII) oder (IX) oder einem pharmazeutisch annehmbaren Salz dieser, wie in einem der Ansprüche 1, 3 und 4 definiert, zur Verwendung als Medikament.

10. Verbindungen oder pharmazeutisch annehmbare Salze dieser gemäß Anspruch 9, wobei die Verbindung der Formel (I) oder ein pharmazeutisch annehmbares Salz dieser mit einer Einheitsdosis von 1 bis 120 mg verabreicht wird.

11. Verbindungen oder pharmazeutisch annehmbare Salze dieser gemäß Anspruch 9 oder 10, wobei die Verbindung der Formel (VII), (VIII) oder (IX) oder ein pharmazeutisch annehmbares Salz dieser mit einer Einheitsdosis von 1 bis 240 mg verabreicht wird.

12. Verbindungen oder pharmazeutisch annehmbare Salze dieser gemäß einem der Ansprüche 9 bis 11, wobei die Verbindung der Formel (I) oder ein pharmazeutisch annehmbares Salz dieser auf intravenösem, oralem oder intramuskulärem Weg verabreicht wird.

13. Verbindungen oder pharmazeutisch annehmbare Salze dieser gemäß einem der Ansprüche 9 bis 12, wobei die Verbindung der Formel (VII), (VIII) oder (IX) oder ein pharmazeutisch annehmbares Salz dieser auf oralem, Injektions- oder lokalem Weg verabreicht wird.

14. Verbindungen oder pharmazeutisch annehmbare Salze dieser gemäß einem der Ansprüche 9 bis 13, um Analgesie zu induzieren oder zur Verwendung in der Prävention oder der Behandlung von Schmerz.

15. Produkte, enthaltend:
- wenigstens eine Verbindung der allgemeinen Formel (I) oder ein pharmazeutisch annehmbares Salz dieser, wie in einem der Ansprüche 1, 2 und 4 definiert, und
- wenigstens eine Verbindung der Formel (VII), (VIII) oder (IX) oder ein pharmazeutisch annehmbares Salz dieser, wie in einem der Ansprüche 1, 3 und 4 definiert,
als Kombinationsprodukt für eine gemeinsame oder getrennte Verwendung, um Analgesie zu induzieren oder um Schmerz zu verhindern oder zu behandeln.

16. Produkte gemäß Anspruch 15, umfassend eine Einheitsdosis von 1 bis 120 mg der Verbindung der Formel (I) oder eines pharmazeutisch annehmbaren Salzes dieser.

17. Produkte gemäß Anspruch 15 oder 16, umfassend eine Einheitsdosis von 1 bis 240 mg der Verbindung der Formel (VII), (VIII) oder (IX) oder eines pharmazeutisch annehmbaren Salzes dieser.

18. Produkte gemäß einem der Ansprüche 15 bis 17, wobei die Verbindung der Formel (I) oder ein pharmazeutisch annehmbares Salz dieser in einer Form ist, die auf intravenösem, oralem oder intramuskulärem Weg verabreichbar ist.

19. Produkte gemäß einem der Ansprüche 15 bis 18, wobei die Verbindung der Formel (VII), (VIII) oder (IX) oder ein pharmazeutisch annehmbares Salz dieser in einer Form ist, die auf oralem, Injektions- oder lokalem Weg verabreichbar ist.

## Claims

1. A pharmaceutical composition comprising as active substances:
a) at least one compound of the following general formula (I): 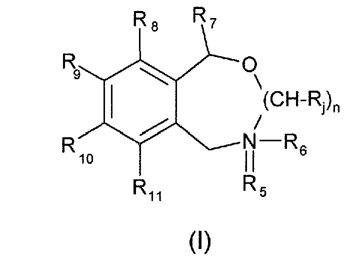 wherein:
- R₅ represents O or no group;
- R₆ represents H or an alkyl group containing from 1 to 6 carbon atoms;
- n represents an integer from 2 to 4;
- j represents an integer ranging from 1 to n;
- Rⱼ, which is identical or different for each substituted carbon, represents H or an alkyl group containing from 1 to 6 carbon atoms;
- R₇ represents a phenyl group optionally substituted by one or more identical or different groups selected from the list comprising H, an alkyl group containing from 1 to 6 carbon atoms, an alkoxy group containing from 1 to 6 carbon atoms, a trifluoromethyl group, or a halogen atom;
- R₈, R₉, R₁₀, R₁₁, which are identical or different, represent H, an alkyl group containing from 1 to 6 carbon atoms, an alkoxy group containing from 1 to 6 carbon atoms, a trifluoromethyl group, or a halogen atom;
or a pharmaceutically acceptable salt of that compound; and
b) at least one compound of one of the following formulae (VII), (VIII), and (IX): 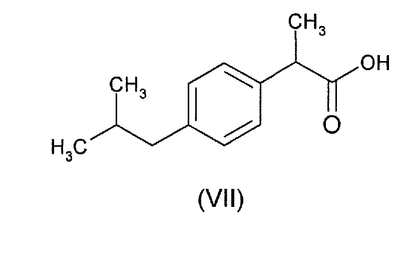 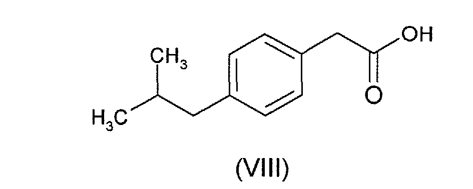 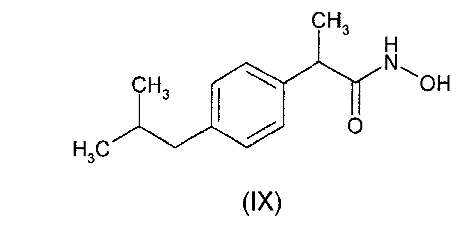 or a pharmaceutically acceptable salt of that compound;
which are optionally combined with one or more pharmaceutically acceptable excipients.

2. The pharmaceutical composition according to claim 1, wherein the compound of formula (I) is represented by one of the following formulae (II), (III) and (IV):

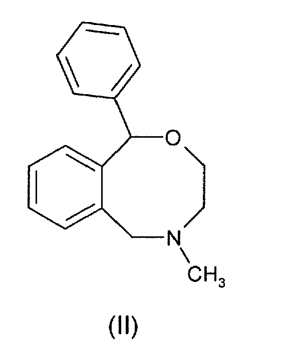

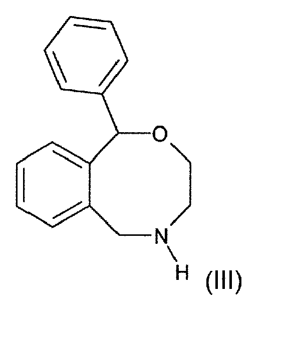

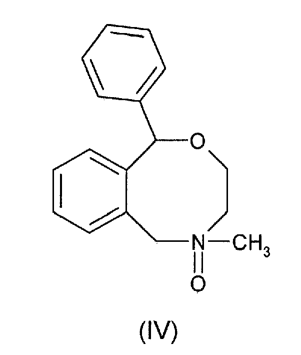

3. The pharmaceutical composition according to claim 1 or 2, wherein the compound of formula (VII) is represented by the following formula (X):

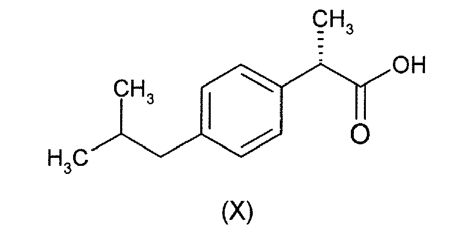

4. The pharmaceutical composition according to any one of claims 1 to 3, comprising:
a) nefopam hydrochloride, and
b) ibuprofen.

5. The pharmaceutical composition according to any one of claims 1 to 4, comprising a unit dose of from 1 to 120 mg of the compound of formula (I) or of a pharmaceutically acceptable salt thereof.

6. The pharmaceutical composition according to any one of claims 1 to 5, comprising a unit dose of from 1 to 2400 mg of the compound of formula (VII), (VIII) or (IX), or of a pharmaceutically acceptable salt thereof.

7. The pharmaceutical composition according to any one of claims 1 to 6, suitable for administration by the oral, injectable or local route.

8. The pharmaceutical composition according to any one of claims 1 to 7, for inducing analgesia or for its use in the prevention or treatment of pain.

9. A Compound of the general formula (I), or a pharmaceutically acceptable salt thereof, as defined in any one of claims 1, 2 and 4, in combination with the compound of formula (VII), (VIII) or (IX), or a pharmaceutically acceptable salt thereof, as defined in any one of claims 1, 3 and 4, for their use as a medicament.

10. The compounds or pharmaceutically acceptable salts thereof according to claim 9, wherein the compound of formula (I), or a pharmaceutically acceptable salt thereof, is administered at a unit dose of from 1 to 120 mg.

11. The compounds or pharmaceutically acceptable salts thereof according to claim 9 or 10, wherein the compound of formula (VII), (VIII) or (IX), a pharmaceutically acceptable salt thereof, is administered at a unit dose of from 1 to 2400 mg.

12. The compounds or pharmaceutically acceptable salts thereof according to any one of claims 9 to 11, wherein the compound of formula (I), or a pharmaceutically acceptable salt thereof, is administered by the intravenous, oral, or intramuscular route.

13. The compounds or pharmaceutically acceptable salts thereof according to any one of claims 9 to 12, wherein the compound of formula (VII), (VIII) or (IX), or a pharmaceutically acceptable salt thereof, is administered by the oral, injectable or local route.

14. The compounds or pharmaceutically acceptable salts thereof according to any one of claims 9 to 13, for inducing analgesia or for their use in the prevention or treatment of pain.

15. Products containing:
- at least one compound of the general formula (I), or a pharmaceutically acceptable salt thereof, as defined in any one of claims 1, 2 and 4, and
- at least one compound of formula (VII), (VIII) or (IX), or a pharmaceutically acceptable salt thereof, as defined in any one of claims 1, 3 and 4,
as a combination product for use together or separately in order to induce analgesia or in order to prevent or treat pain.

16. The products according to claim 15, comprising a unit dose of from 1 to 120 mg of the compound of formula (I), or of a pharmaceutically acceptable salt thereof.

17. The products according to claim 15 or 16, comprising a unit dose of from 1 to 2400 mg of the compound of formula (VII), (VIII) or (IX), or of a pharmaceutically acceptable salt thereof.

18. The products according to any one of claims 15 to 17, wherein the compound of formula (I), or a pharmaceutically acceptable salt thereof, is in a form administrable by the intravenous, oral or intramuscular route.

19. The products according to any one of claims 15 to 18, wherein the compound of formula (VII), (VIII) or (IX), or a pharmaceutically acceptable salt thereof, is in a form administrable by the oral, injectable or local route.
